# EUROPEAN PATENT SPECIFICATION

(11) **EP 2 623 216 B1**
(45) Date of publication and mention of the grant of the patent: **24.08.2022**
(21) Application number: 13153429.9
(22) Date of filing: 31.01.2013
(51) Int. Cl.: B06B 1/06, G10K 11/00

(54) **Ultrasonic probe and manufacturing method thereof**
Ultraschallsonde und Herstellungsverfahren dafür
Sonde ultrasonore et son procédé de fabrication

(30) Priority: 31.01.2012 KR 20120010064
(43) Date of publication of application: 07.08.2013
(73) Proprietor: Samsung Medison Co., Ltd., Gangwon-do 250-870 (KR)
(72) Inventor: Jin, Gil-ju, Seoul (KR)
(74) Representative: Schmid, Wolfgang

(56) References cited:
- EP-A2- 0 637 470
- WO-A2-02/40184
- US-A1- 2008 098 816
- US-A1- 2008 200 812
- US-B1- 6 625 854

## Description

### BACKGROUND

### 1. Field

Embodiments of the present disclosure relate to an ultrasonic probe configured to generate an image of an inside of a subject by using an ultrasonic wave.

### 2. Description of the Related Art

An ultrasonic diagnostic apparatus is an apparatus configured to scan an ultrasonic signal toward a desired portion at an inside of a body of a subject from a surface of a body, and to obtain an image through a non-invasive measurement with respect to a cross section of a soft tissue or a blood flow by using the information of the ultrasonic signal which is reflected, i.e., an ultrasonic echo signal. The apparatus, when compared to other image diagnostic apparatuses such as an x-ray diagnostic apparatus, an x-ray CT scanner (a Computerized Tomography scanner), a MRI (a Magnetic Resonance Image), or a nuclear medicine diagnostic apparatus, is small-sized and inexpensive, while capable of displaying an image in real time. Further, since no radiation exposure of x-ray is present, a high level of safety is provided. Thus, the apparatus is widely used in the diagnosis of a heart, a stomach, and a urinary system, as well as in the diagnosis of gynecology.

Particularly, the ultrasonic diagnostic apparatus includes an ultrasonic probe configured to transmit an ultrasonic signal to a subject to obtain an ultrasonic image of the subject, and to receive the ultrasonic echo signal reflected from the subject.

The ultrasonic probe includes a piezo-electric layer configured to reciprocally change an electrical signal and an acoustic signal while a piezo-electric material is vibrated, a matching layer configured to decrease a difference of acoustic impedance between the piezo-electric layer and a subject so that the ultrasonic wave generated at the piezo-electric layer may be delivered to the subject as much as possible, a lens layer configured to gather the ultrasonic wave proceeding toward a front of the piezo-electric layer to a particular point, a transducer module having an acoustic absorption layer, that is, a backing layer, that prevents an image distortion by blocking the ultrasonic wave from proceeding toward a rear of the piezo-electric layer, a case having an upper portion thereof open, and a cover configured to make contact with a surface of the subject while being coupled to the upper portion of the case having an opening thereof.

The ultrasonic diagnostic apparatus is configured to obtain a cross-sectional image of a subject by scanning an ultrasonic wave at an inside of the subject, and detecting the reflection of the ultrasonic wave. The ultrasonic diagnostic apparatus includes a mechanical scanning method configured to scan an ultrasonic wave by moving an ultrasonic probe in a mechanical manner, and an electronic scanning method configured to scan an ultrasonic wave by a control of the electronic change and the delayed time by using an array oscillator, e.g., an ultrasonic transducer array.

A conventional ultrasonic diagnostic apparatus is generally configured to scan an ultrasonic beam to an inside of a single surface, and thus is composed of a system configured to display a cross section, that is, a plane surface image. However, in recent years, an attempt to obtain three-dimensional information by collecting a diagnostic image while moving an ultrasonic probe, which is an ultrasonic transmission/reception unit of an ultrasonic diagnostic apparatus, has been frequently made, and displaying of a three-dimensional image with respect to an ultrasonic diagnostic apparatus is expected to lead to a possibility of a new diagnosis.

Particularly, a two-dimensional ultrasonic transducer array and a two-dimensional array system provided with an objective to obtain three-dimensional image information, by arranging ultrasonic transducer elements (oscillators) that compose the piezo-electric layer of the ultrasonic probe in a two-dimensional manner and by scanning a beam at a diagnostic domain in a three-dimensional manner through the control of the electronic change and the delayed time, has been researched.

By using the ultrasonic transducers employed with the two-dimensional array system, when compared to a system configured to mechanically move the ultrasonic transducer, a collection of three-dimensional information may be able to be performed within a short period of time, and the real-time processing performance may be remarkably improved.

When manufacturing the two-dimensional transducer array used at the two-dimensional array system, the ultrasonic transducer elements are arranged in a two-dimensional manner while having a microscopic distance between the ultrasonic transducer elements so that an ultrasonic wave may be deflected toward a predetermined direction. At this time, from each of the ultrasonic transducer elements arranged in a two-dimensional manner, an electrode lead wire electrically connected is needed to be drawn.

However, since the number of the ultrasonic transducer elements arranged at the two-dimensional transducer array is in thousands of units, the drawing of the electrode lead wire from the each of the ultrasonic transducer elements is practically difficult, and even in a case when the electrode lead wire is drawn from the each of the ultrasonic transducer elements, a large number of electrode lead wires being drawn may be tangled at an inside of a narrow space of the piezo-electric layers, and thus a configuration of a circuit is complicated.

US 2008/098816 A1 discloses an ultrasonic probe comprising an acoustic backing layer made of a composite resin material including a resin and a plurality of bonded fibers containing the resin.

EP 0 637 470 A2 discloses an acoustic transducer for transmitting acoustic wave energy in response to an electrical signal and for converting the received acoustic wave energy into an electric signal.

WO 02/40184 A2 describes a two-dimensional ultrasonic transducer array stack which has a backing block of acoustically absorbent material formed of alternating plates of backing material and flex circuits adhesively bonded together.

US 6 625 854 B1 discloses an acoustic backing element including a glass fiber epoxy composite planar substrate to the outer major surfaces of which are applied electrically conductive material.

US 2008/200812 A1 discloses an ultrasonic probe that can be relatively easily manufactured and has a desired ultrasonic radiation surface.

### SUMMARY

The invention is defined by claim 1 or 2.

Regarding the method of manufacturing an ultrasonic probe the invention is defined by claim 5 or 6.

Additional aspects of the disclosure will be set forth in part in the description which follows and, in part, will be obvious from the description, or may be learned by practice of the disclosure.

In some examples at least an upper surface or a lower surface of the member may be acoustically connected to the ultrasonic transducer array.

In some examples, the plurality of members may be adhesively attached to each other by using same material as material of the member, and are arranged in a two-dimensional manner.

In some example, the method may be achieved by further performing the following step. At least an upper surface or a lower surface of the member may be acoustically connected to the ultrasonic transducer array.

In some example, the arranging of the plurality of members in a two-dimensional manner may include adhesively attaching the plurality of members each other by using same material as material of the member.

### BRIEF DESCRIPTION OF THE DRAWINGS

These and/or other aspects of the disclosure will become apparent and more readily appreciated from the following description of the embodiments, taken in conjunction with the accompanying drawings of which:

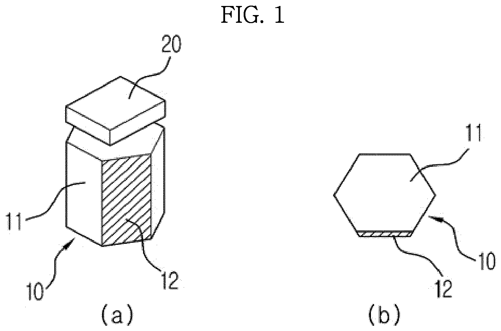

FIG. 1 illustrates an exemplary perspective view and an exemplary cross-sectional view of a backing element of an ultrasonic probe in accordance with the first example not forming part of the invention.

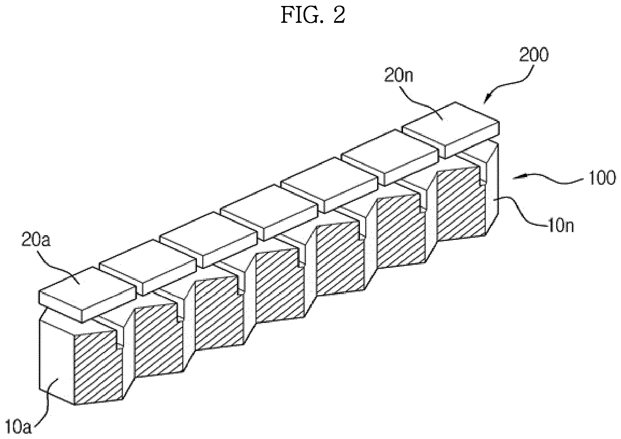

FIG. 2 is an exemplary perspective view of a backing layer of the ultrasonic probe formed by arranging the backing elements of the ultrasonic probe in a one-dimensional manner in accordance with the first example.

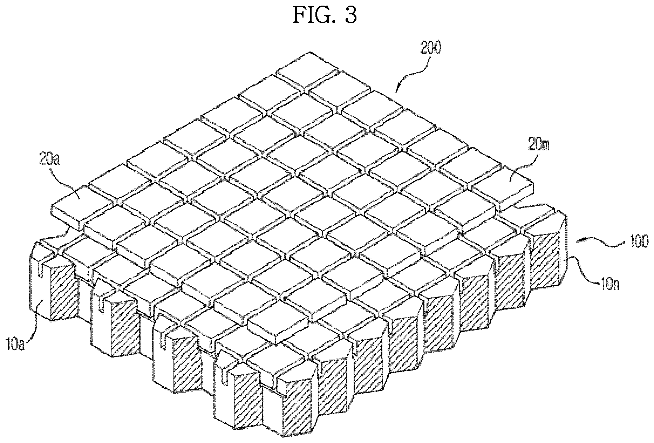

FIG. 3 is an exemplary perspective view of a backing layer of the ultrasonic probe formed by arranging the backing elements of the ultrasonic probe in a two-dimensional manner in accordance with the first example.

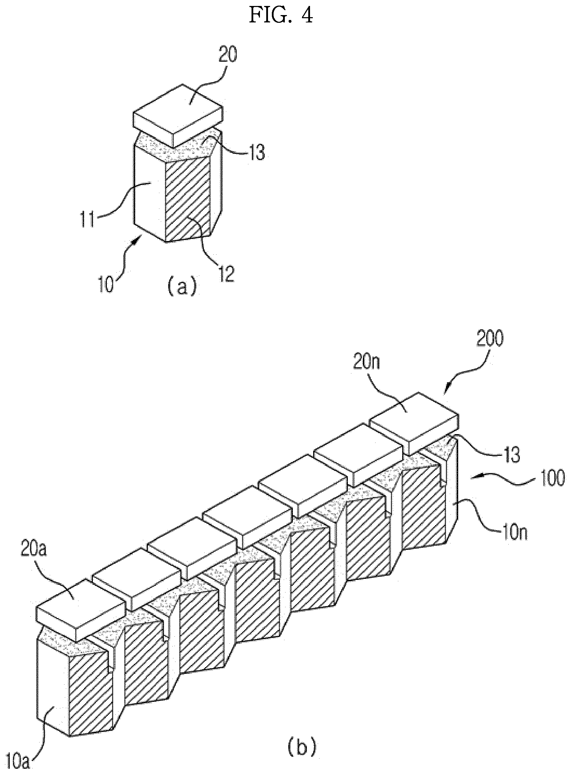

FIG. 4(a) illustrates an exemplary perspective view of a backing element of an ultrasonic probe in accordance with the second example not forming part of the invention of the present disclosure, and FIG. 4(b) illustrates an exemplary perspective view of a backing layer of the ultrasonic probe formed by arranging the backing elements of the ultrasonic probe in a one-dimensional manner in accordance with the second example.

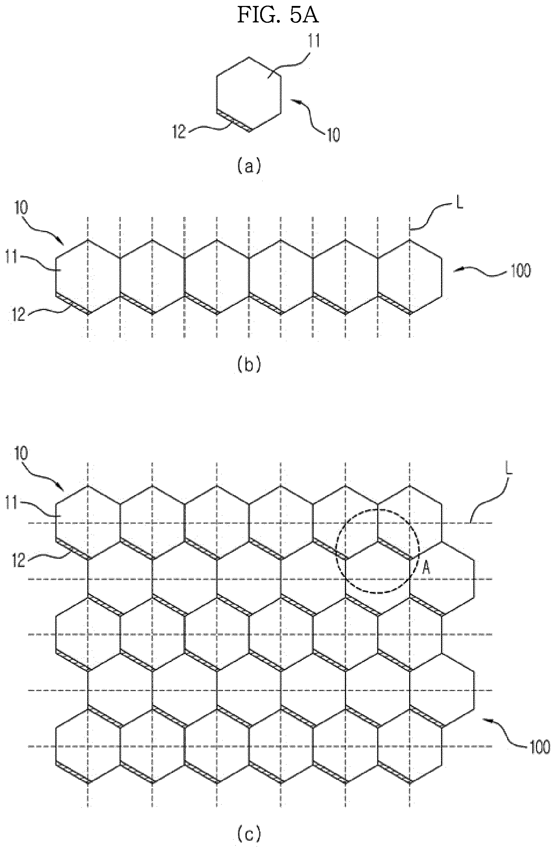

FIG. 5A(a) is an exemplary cross-sectional view provided to describe the backing element of the ultrasonic probe in accordance with the first example in a case when a conductive trace is formed only at one side surface of the member having a shape of a polygonal (hexagonal) column that forms the backing element of the ultrasonic probe, FIG. 5A(b) illustrates the backing layer of the ultrasonic probe formed by arranging the backing elements of the ultrasonic probe in a one-dimensional manner, and FIG. 5A(c) illustrates the backing layer of the ultrasonic probe formed by arranging the backing elements of the ultrasonic probe in a two-dimensional manner.

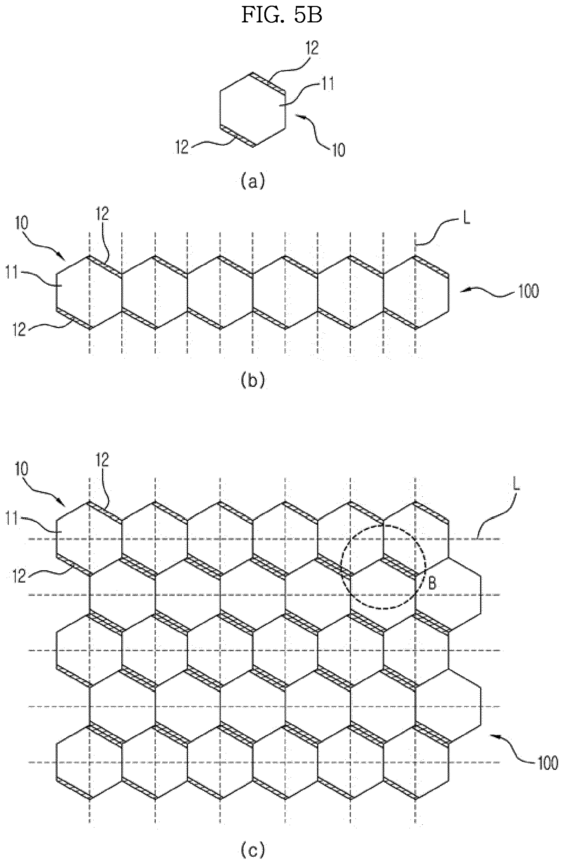

FIG. 5B(a) is an exemplary cross-sectional view provided to describe the backing element of the ultrasonic probe in accordance with an embodiment of the present disclosure in a case when conductive traces are formed at two side surfaces of the member having a shape of a polygonal (hexagonal) column that forms the backing element of the ultrasonic probe, FIG. 5B(b) illustrates the backing layer of the ultrasonic probe formed by arranging the backing elements of the ultrasonic probe in a one-dimensional manner, and FIG. 5B(c) illustrates the backing layer of the ultrasonic probe formed by arranging the backing elements of the ultrasonic probe in a two-dimensional manner.

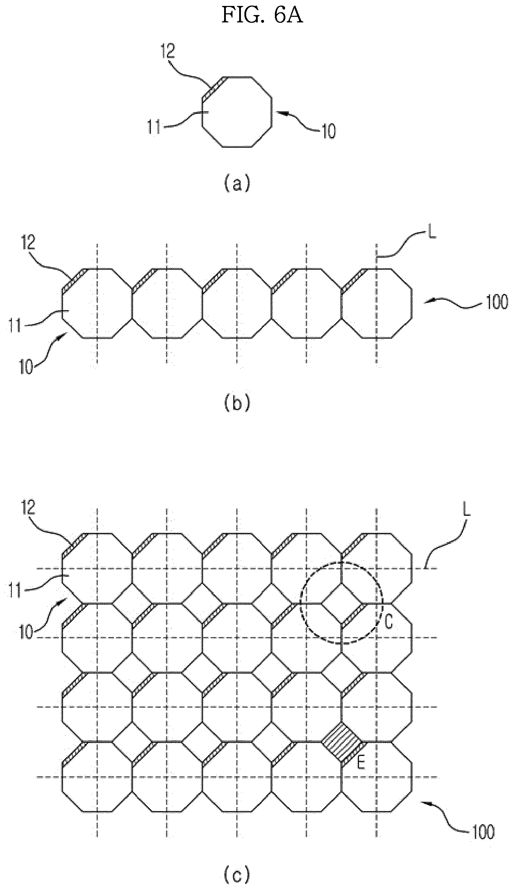

FIG. 6A(a) is an exemplary cross-sectional view provided to describe a backing element of an ultrasonic probe in accordance with the third example not forming part of the invention in a case when a conductive trace is formed only at one side surface of the member having a shape of a polygonal (octagonal) column that forms the backing element of the ultrasonic probe, FIG. 6A(b) illustrates the backing layer of the ultrasonic probe formed by arranging the backing elements of the ultrasonic probe in a one-dimensional manner, and FIG. 6A(c) illustrates the backing layer of the ultrasonic probe formed by arranging the backing elements of the ultrasonic probe in a two-dimensional manner.

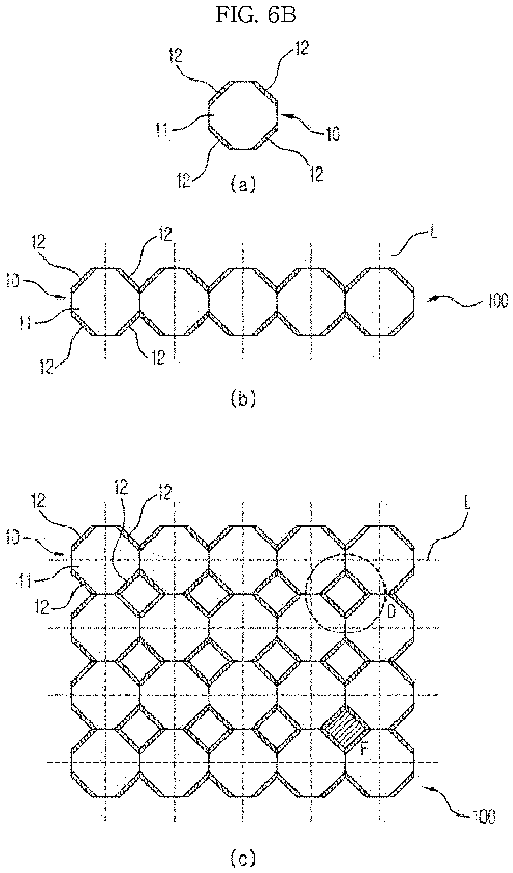

FIG. 6B(a) is an exemplary cross-sectional view provided to describe the backing element of the ultrasonic probe in accordance with an embodiment of the present disclosure in a case when conductive traces are formed at four side surfaces of the member having a shape of a polygonal (octagonal) column that forms the backing element of the ultrasonic probe, FIG. 6B(b) illustrates the backing layer of the ultrasonic probe formed by arranging the backing elements of the ultrasonic probe in a one-dimensional manner, and FIG. 6B(c) illustrates the backing layer of the ultrasonic probe formed by arranging the backing element of the ultrasonic probe in a two-dimensional manner.

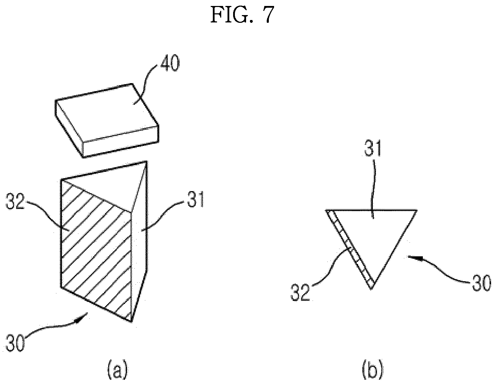

FIG. 7(a) illustrates an exemplary perspective view and FIG. 7(b) illustrates an exemplary cross-sectional view of a backing element of an ultrasonic probe in accordance with the fourth example not forming part of the invention.

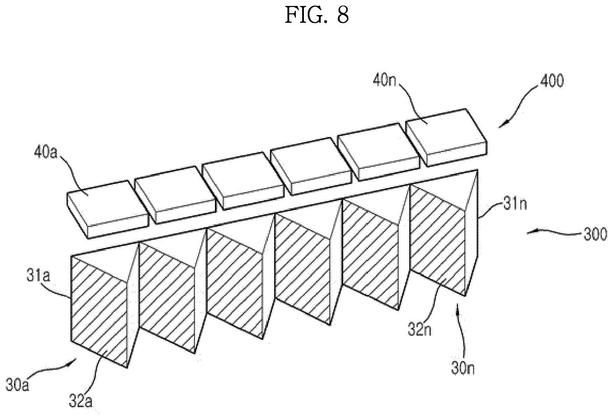

FIG. 8 is an exemplary perspective view of a backing layer of the ultrasonic probe formed by arranging the backing elements of the ultrasonic probe in a one-dimensional manner in accordance with the fourth example.

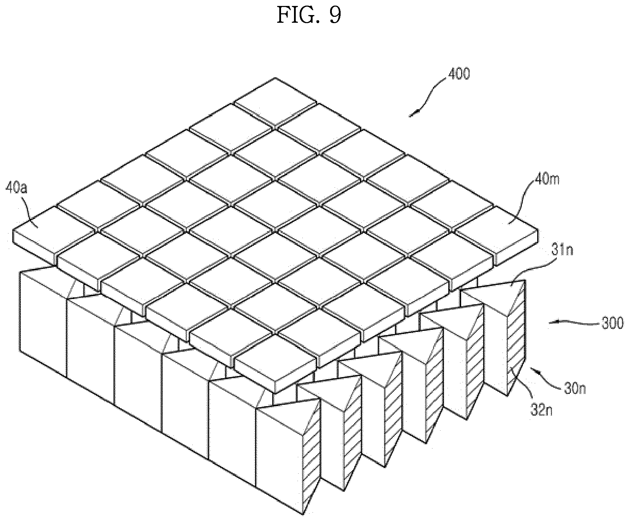

FIG. 9 is an exemplary perspective view of a backing layer of the ultrasonic probe formed by arranging the backing elements of the ultrasonic probe in a two-dimensional manner in accordance with the fourth example.

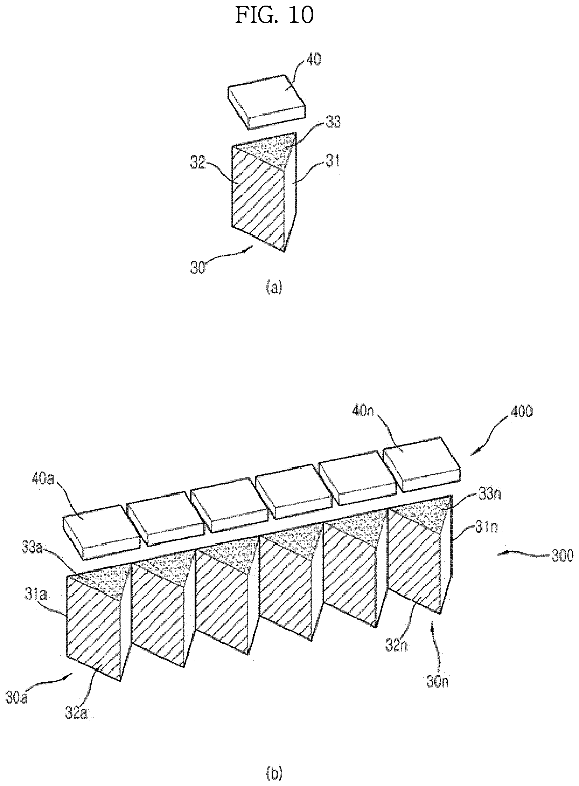

FIG. 10(a) illustrates an exemplary perspective view of a backing element of an ultrasonic probe in accordance with the fifth example not forming part of the invention, and FIG. 10(b) illustrates an exemplary perspective view of a backing layer of the ultrasonic probe formed by arranging the backing elements of the ultrasonic probe in a one-dimensional manner in accordance with the fifth example.

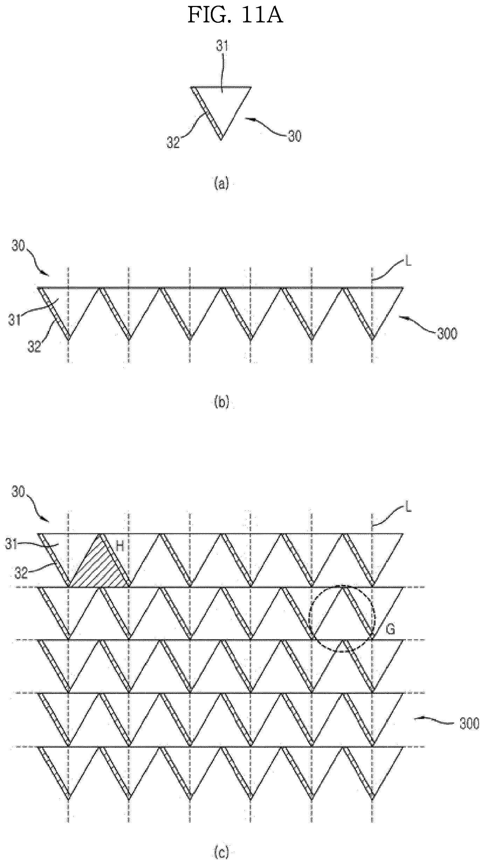

FIG. 11A(a) is an exemplary cross-sectional view provided to describe the backing element of the ultrasonic probe in accordance with the fourth example in a case when a conductive trace is formed only at one side surface of the member having a shape of a polygonal (triangular) column that forms the backing element of the ultrasonic probe, FIG. 11A(b) illustrates the backing layer of the ultrasonic probe formed by arranging the backing elements of the ultrasonic probe in a one-dimensional manner, and FIG. 11A(c) illustrates the backing layer of the ultrasonic probe formed by arranging the backing elements of the ultrasonic probe in a two-dimensional manner.

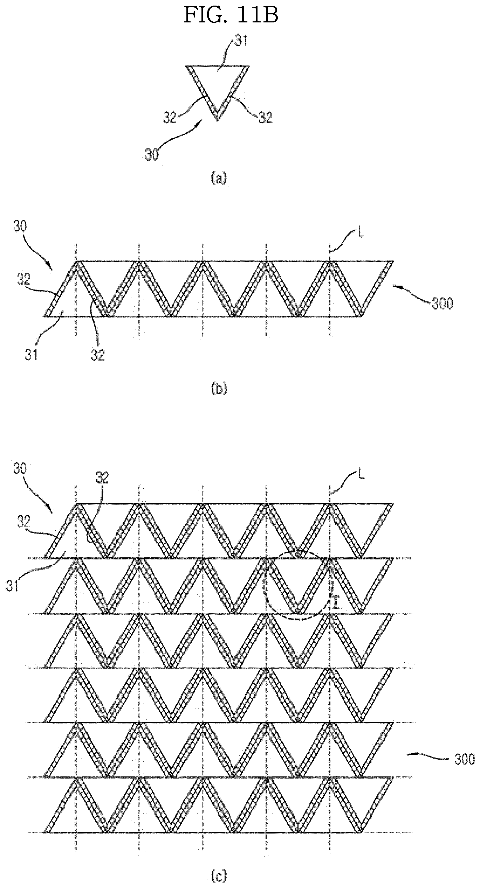

FIG. 11B(a) is an exemplary cross-sectional view provided to describe the backing element of the ultrasonic probe in accordance with the fourth example in a case when conductive traces are formed at two side surfaces of the member having a shape of a polygonal (triangular) column that forms the backing element of the ultrasonic probe, FIG. 11B(b) illustrates the backing layer of the ultrasonic probe formed by arranging the backing elements of the ultrasonic probe in a one-dimensional manner, and FIG. 11B(c) illustrates the backing layer of the ultrasonic probe formed by arranging the backing elements of the ultrasonic probe in a two-dimensional manner.

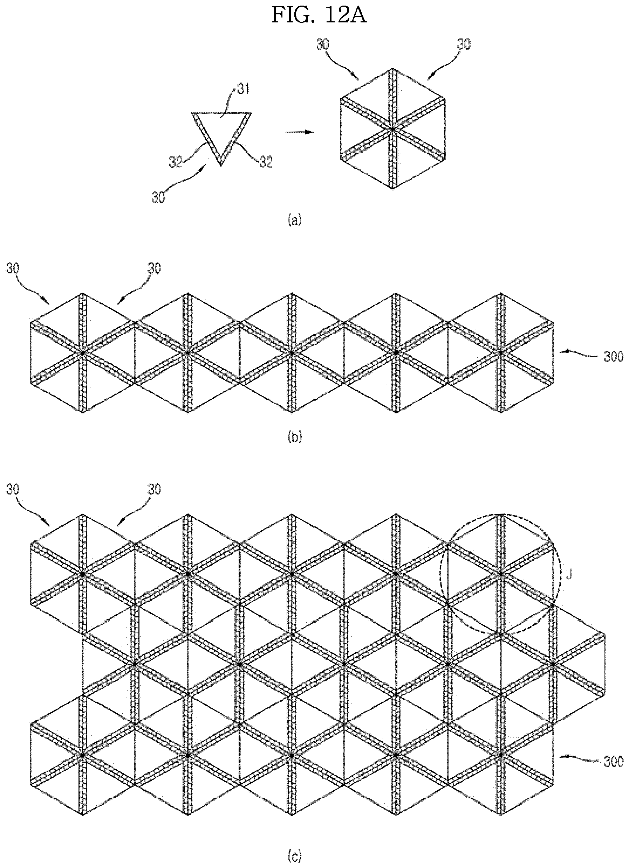

FIG. 12A(a) is an exemplary cross-sectional view provided to describe the backing element of the ultrasonic probe not forming part of the invention in a case when conductive traces are formed at two side surfaces of the member having a shape of a triangular column, FIG. 12A(b) illustrates the backing element of the ultrasonic probe having a shape of a hexagonal column that is formed by a combination of six of the backing elements, the backing layer of the ultrasonic probe formed by arranging the hexagonal column shape backing elements in a one-dimensional manner, and FIG. 12A(c) illustrates the backing layer of the ultrasonic probe formed by arranging the hexagonal column shape backing elements in a two-dimensional manner.

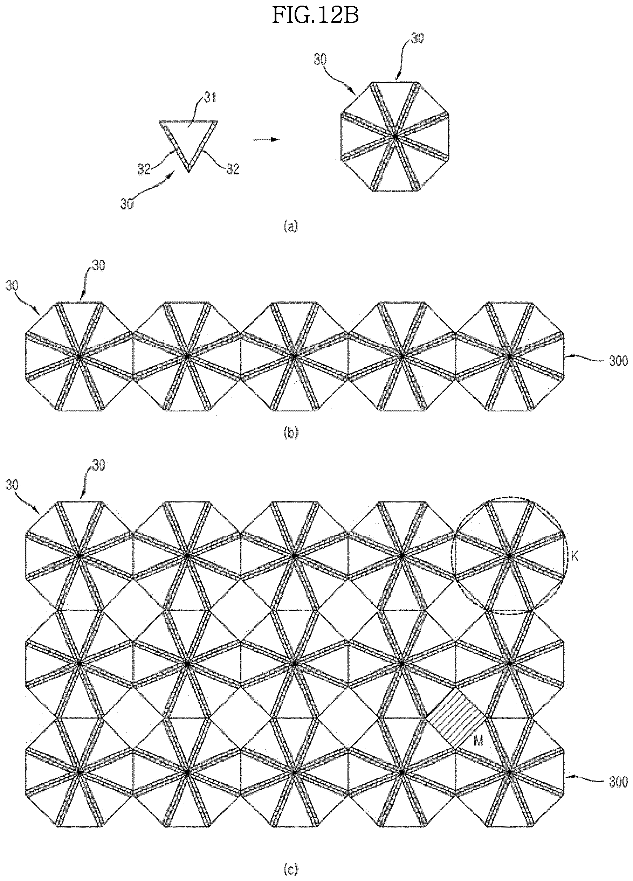

FIG. 12B(a) is an exemplary cross-sectional view provided to describe the backing element of the ultrasonic probe not forming part of the invention in a case when conductive traces are formed at two side surfaces of the member having a shape of a triangular column, FIG. 12B(b) illustrates the backing element of the ultrasonic probe having a shape of an octagonal column that is formed by a combination of eight of the backing elements, the backing layer of the ultrasonic probe formed by arranging the octagonal column shape backing elements in a one-dimensional manner, and FIG. 12B(c) illustrates the backing layer of the ultrasonic probe formed by arranging the octagonal column shape backing elements in a two-dimensional manner.

### DETAILED DESCRIPTION

Reference will now be made in detail to the embodiments of the present disclosure, examples of which are illustrated in the accompanying drawings, wherein like reference numerals refer to like elements throughout.

FIG. 1 illustrates a perspective view and a cross-sectional view of a backing element of an ultrasonic probe in accordance with the first example.

As illustrated in FIG. 1(a), a backing element 10 of an ultrasonic probe in accordance with the example includes a member 11 having a shape of a polygonal column and a conductive trace 12 formed at one side surface of the member 11. In FIG. 1, as the member 11 having a shape of a polygonal column, the member having a shape of a hexagonal column from various polygonal columns is provided as an example.

The backing element 10, as illustrated in FIG. 1(a), is a basic component of a backing layer that is configured to absorb an ultrasonic wave, which is radiated toward a rear of a piezo-electric layer by the resonance of a piezo-electric element (oscillator) that composes the piezo-electric layer at an inside of a transducer module. Here, the member 11 having a shape of a polygonal column is manufactured by a synthetic material composed of epoxy resin, tungsten, and rubber powder, and other than the synthetic material. Any material suitable for absorbing rear sound caused by the resonance of piezo-electric material may be used in manufacturing the member 11 having a shape of a polygonal column.

As illustrated in FIG. 1(a), at one side surface of the member 11 having a shape of a hexagonal column while provided with six units of side surfaces thereof, a conductive trace 12 is formed. Here, the forming of the conductive trace 12 is referred to as adding (plastering) an electrical contact material on a side surface of the member 11 having a shape of a polygonal column by using various adjustment synthesis techniques. When forming the conductive trace 12, as the electrical contact material, metals, conductive graphite, or conductive ink may be used, and as a method of adding electrical contact material on a side surface of the member 11 having a shape of a polygonal column, a technique such as laser scribing, planting, deposition, or printing may be applied.

The backing element 10 is electrically and acoustically connected to one ultrasonic transducer element 20. The ultrasonic transducer element 20 is generally composed of PZT (Lead Zirconate Titanate Ceramics) or a MUT (Micro-machined Ultrasonic Transducer). The electrical and acoustic connection between the backing element 10 and the ultrasonic transducer element 20 will be described in more detail. The member 11 is acoustically connected to the ultrasonic transducer element 20 by making contact with the one ultrasonic transducer element 20, so that the member 11 weakens or absorbs acoustic energy that is radiated from a rear surface of the ultrasonic transducer element 20. In addition, the conductive trace 12 formed at one side surface of the member 11 having a shape of a polygonal column is electrically connected to the ultrasonic transducer element 20, thereby electrically connects the backing element 10 to the ultrasonic transducer element 20.

FIG. 1(b) shows a cross section of the backing element 10, and illustrates an image of the conductive trace 12 formed only at one side surface of the member 11 having a shape of a hexagonal column provided with six units of side surfaces thereof.

FIG. 2 is a perspective view of a backing layer of the ultrasonic probe formed by arranging the backing elements of the ultrasonic probe in a one-dimensional manner in accordance with the first example.

The backing element 10, which is same as the backing element 10 illustrated in FIG 1(a), is provided in plural units (an 'n' number of the backing elements 10). By arranging a plurality of backing elements 10a to 10n in a one-dimensional manner, that is, in a linear manner, a backing layer 100 is formed as illustrated in FIG. 2.

As illustrated in FIG. 2, at the backing layer 100 formed by arranging the plurality (the 'n' number) of the backing elements 10a to 10n in a one-dimensional manner, a one-dimensional ultrasonic transducer array 200 formed by arranging a plurality (an 'n' number) of ultrasonic transducer elements 20a to 20n in a one-dimensional manner is electrically and acoustically connected. The electrical and acoustic connection between the backing layer 100 and the one-dimensional ultrasonic transducer array 200 will be described in more detail. The plurality of members having a shape of a polygonal column that compose the backing layer 100 are acoustically connected to the one-dimensional ultrasonic transducer array 200 by making contact with the one-dimensional ultrasonic transducer array 200, so that the plurality of members weakens or absorbs acoustic energy that is radiated from a rear surface of the one-dimensional ultrasonic transducer array 200. In addition, the conductive traces each formed at each of the members having a shape of a polygonal column are electrically connected to the plurality (the 'n' number) of the ultrasonic transducer elements 20a to 20n that composes the one-dimensional ultrasonic transducer array 200, respectively, thereby electrically connect the backing elements to the ultrasonic transducer elements 20a to 20n. Although not illustrated in FIG. 2, the backing layer 100 of the ultrasonic probe is mounted on a printed circuit board (not shown), and the conductive traces each formed at each of the members having a shape of a polygonal column forming the backing layer 100 are electrically connected to conductive wires formed at the printed circuit board, and electrically connect each of the ultrasonic transducer elements 20a to 20n.

FIG. 3 is a perspective view of a backing layer of the ultrasonic probe formed by arranging the backing elements of the ultrasonic probe in a two-dimensional manner in accordance with the first example.

The backing element 10, which is same as the backing element 10 illustrated in FIG 1(a), is provided in plural units (an 'n' number of the backing elements 10). By arranging a plurality of backing elements 10a to 10n in a two-dimensional manner, that is, in a matrix manner, the backing layer 100 is formed as illustrated in FIG. 3.

As illustrated in FIG. 3, at the backing layer 100 formed by arranging the plurality (the 'n' number) of the backing elements 10a to 10n in a two-dimensional manner, a two-dimensional ultrasonic transducer array 200 formed by arranging a plurality (an 'm' number) of ultrasonic transducer elements 20a to 20m in a two-dimensional manner is electrically and acoustically connected. The electrical and acoustic connection between the backing layer 100 and the two-dimensional ultrasonic transducer array 200 will be described in detail. The plurality of members having a shape of a polygonal column that compose the backing layer 100 are acoustically connected to the two-dimensional ultrasonic transducer array 200 by making contact with the two-dimensional ultrasonic transducer array 200, so that the plurality of members weakens or absorbs acoustic energy that is radiated from a rear surface of the two-dimensional ultrasonic transducer array 200. In addition, the conductive traces each formed at each of the members having a shape of a polygonal column are electrically connected to the plurality (the 'm' number) of the ultrasonic transducer elements 20a to 20m, respectively, thereby electrically connect the plurality of members to the ultrasonic transducer elements 20a to 20m. Although not illustrated on FIG. 3, the backing layer 100 of the ultrasonic probe is mounted on a printed circuit board (not shown), and the conductive traces each formed at each of the members having a shape of a polygonal column forming the backing layer 100 are electrically connected to conductive wires formed at the printed circuit board, and electrically connect each of the ultrasonic transducer elements 20a to 20m.

FIGS. 4(a) and 4(b) illustrate a perspective view of a backing element of an ultrasonic probe in accordance with the second example, and a perspective view of a backing layer of the ultrasonic probe formed by arranging the backing elements of the ultrasonic probe in a one-dimensional manner in accordance with the second example, respectively.

As illustrated in FIG. 4(a), the backing element 10 of an ultrasonic probe in accordance with the second example is different from the backing element 10 illustrated on (a) of FIG. 1. In the second example, an electrode layer 13 is further formed at an upper surface or at a lower surface of the member 11 that is acoustically connected to the ultrasonic transducer element 20. When the electrode layer 13 is formed at an upper surface or at a lower surface of the member 11 acoustically connected to the ultrasonic transducer element 20, the electrical connection between the ultrasonic transducer element 20 and the conductive trace 12 formed at a side surface of the member 11 having a shape of a polygonal column may be further improved.

The backing element 10, which is same as the backing element 10 illustrated in FIG 4(a), is provided in plural units (an 'n' number of the backing element 10), and by arranging a plurality of backing elements 10a to 10n in a one-dimensional manner, that is, in a linear manner, the backing layer 100 is formed as illustrated in FIG. 4(b).

With respect to the structure of a circuit of the backing layer 100 of the ultrasonic probe formed by arranging the backing elements in a one-dimensional manner in accordance with the second example as illustrated in FIG. 4(b), other than the structure of the electrode layer 13 formed at an upper surface or at a lower surface of the member 11 having a shape of a polygonal column, since all other components are same as the structure of the backing layer 100 of an ultrasonic probe formed by arranging the backing elements of an ultrasonic probe in a one-dimensional manner in accordance with the second example as illustrated in FIG. 2, the detailed description thereof will be omitted.

FIG. 5A(a) is a cross-sectional view of the backing element 10 having the member 11 having a shape of a polygonal (hexagonal) column and the conductive trace 12 formed at one side surface of the member 11 having a shape of a hexagonal column, FIG. 5A(b) is a cross-sectional view of the backing layer 100 formed by arranging the backing elements 10 illustrated at FIG. 5A(a) in a one-dimensional manner, and FIG. 5A(c) is a cross-sectional view of the backing layer 100 formed by arranging the backing elements 10 illustrated at FIG. 5A(a) in a two-dimensional manner. The straight line illustrated with a dotted line on FIGS. 5A(a) and 5A(b) is referred to as a dicing line 'L'. At a domain (a curvature domain illustrated with dotted line at FIG. 5A(c), that is, the "A" domain) divided by the dicing line 'L', one ultrasonic transducer element is connected.

The case of the forming of the backing layer 100, which is prepared by arranging the members 11 having a shape of a polygonal column in a one-dimensional manner or in a two-dimensional manner while each of the members 11 having a shape of a polygonal column is provided with the conductive trace 12 formed only at one side surface thereof, has been described. Hereinafter, by referring to FIG. 5B, a case of the forming of the backing layer 100, which is prepared by arranging the members 11 having a shape of a polygonal column in a one-dimensional manner or in a two-dimensional manner while each of the members 11 having a shape of a polygonal column is provided with the conductive traces 12 formed at two side surfaces thereof, will be described.

FIG. 5B(a) is a cross-sectional view of the backing element 10 that includes the member 11 having a shape of a polygonal (hexagonal) column and also includes the conductive traces 12 formed at two side surfaces of the member 11 having a shape of a hexagonal column, FIG. 5B(b) is a cross-sectional view of the backing layer 100 formed by arranging the backing elements 10 illustrated at FIG. 5A(a) in a one-dimensional manner, and FIG. 5B(c) is a cross-sectional view of the backing layer 100 formed by arranging the backing elements 10 illustrated at FIG. 5B(a) in a two-dimensional manner. The linear line illustrated with a dotted line on FIGS. 5B(b) and 5B(c) is referred to as a dicing line 'L'. At a domain (a curvature domain illustrated with dotted line at FIG. 5B(c), that is, a "B" domain) divided by the dicing line 'L', one ultrasonic transducer element is connected.

When comparing the case of forming the backing layer 100 by arranging the members 11 having a shape of a hexagonal column in a two-dimensional manner while the each of the members 11 is provided with the conductive trace 12 formed only at one side surface thereof (refer to FIG. 5A(c)) to the case of forming the backing layer 100 by arranging the members 11 having a shape of a hexagonal column in a two-dimensional manner while the each of the members 11 is provided with the conductive trace 12 formed at two side surfaces thereof (refer to FIG. 5B(c)), with respect to the thickness of the conductive trace 12 of the "A" domain illustrated in FIG. 5A(c), the thickness of the conductive trace 12 of the "B" domain illustrated in FIG. 5B(c) becomes thicker by about twice. As such, since the thickness of the conductive trace 12 electrically connected to one ultrasonic transducer element becomes thicker, the electrical characteristic of the conductive trace 12 may be further improved.

FIG. 6A(a) is a cross-sectional view of the backing element 10 having the member 11 having a shape of a polygonal (octagonal) column and the conductive trace 12 formed at one side surface of the member 11 having a shape of an octagonal column, FIG. 6A(b) is a cross-sectional view of the backing layer 100 formed by arranging the backing elements 10 illustrated at FIG. 6A(a) in a one-dimensional manner, and FIG. 6A(c) is a cross-sectional view of the backing layer 100 formed by arranging the backing elements 10 illustrated at FIG. 6A(a) in a two-dimensional manner. The linear line illustrated with a dotted line on FIGS. 6A(b) and 6A(c) is referred to as a dicing line 'L'. At a domain (a curvature domain illustrated with dotted line at FIG. 6A(c), that is, the "C" domain) divided by the dicing line 'L', one ultrasonic transducer element is connected.

FIG. 6B(a) is a cross-sectional view of the backing element 10 having the member 11 having a shape of a polygonal (octagonal) column and the conductive traces 12 formed at four side surfaces of the member 11 having a shape of an octagonal column, FIG. 6B(b) is a cross-sectional view of the backing layer 100 formed by arranging the backing elements 10 illustrated at FIG. 6A(a) in a one-dimensional manner, and FIG. 6B(c) is a cross-sectional view of the backing layer 100 formed by arranging the backing elements 10 illustrated at FIG. 6B(a) in a two-dimensional manner. The linear line illustrated with a dotted line on FIGS. 6B(b) and 6B(c) is referred to as a dicing line 'L'. At a domain (a curvature domain illustrated with dotted line at (c) of FIG. 6B, that is, the "D" domain) divided by the dicing line 'L', one ultrasonic transducer element is connected.

When comparing the case of forming the backing layer 100 by arranging the members 11 having a shape of an octagonal column in a two-dimensional manner while each member 11 is provided with the conductive trace 12 formed only at one side surface thereof (refer to FIG. 6A(c)) to the case of forming the backing layer 100 by arranging the members 11 having a shape of an octagonal column in a two-dimensional manner while each member 11 is provided with the conductive trace 12 formed at four side surfaces thereof (refer to FIG. 6B(c)), with respect to the conductive trace 12 present at the "C" domain illustrated in FIG. 6A(c), the conductive trace 12 present at the "D" domain illustrated in FIG. 6B(c) is greater by about four times. As such, since the amount of the conductive trace 12 electrically connected to one ultrasonic transducer element increases, the electrical characteristic of the conductive trace 12 may be further improved.

Meanwhile, in a case of forming the backing layer 100 by arranging the members 11 having a shape of an octagonal column in a two-dimensional manner while each member 11 is provided with the conductive trace 12 formed only at one side surfaces thereof (refer to FIG. 6A(c)) or in a case of forming the backing layer 100 by arranging the members 11 having a shape of an octagonal column in a two-dimensional manner while each member 11 is provided with the conductive trace 12 formed at four side surfaces thereof (refer to FIG. 6B(c)), at each of an in-between area of the arrangements of the members 11 having a shape of an octagonal column, empty spaces (the "E" domain of FIG. 6A(c) and the "F" domain of FIG. 6B(c)) are formed. The backing layer 100 is needed to perform a function to prevent an image distortion by blocking an ultrasonic wave being proceeded toward a rear of the piezo-electric layer. When an empty space is present at an inside of the backing layer 100, the backing performance of the backing layer 100 may be reduced. Thus, in addition to the case when the empty space is formed between the arrangements of the members having a shape of an octagonal column, as in the case of the two-dimensional arrangement of the members 11 having a shape of an octagonal column, also in a case when a visible empty space is not formed at each of an in-between area of the arrangements of the members having a shape of a hexagonal column, as in the case of the two-dimensional arrangement of the members having a shape of a hexagonal column, the plurality of members provided with various shapes of polygonal column thereof may be adhesively attached to each other by using the same material as the material of the members when arranging the plurality of members provided with various shapes of polygonal column thereof in a one-dimensional manner or in a two-dimensional manner, and thus the valid area of the backing layer 100 may be increased.

FIGS. 7(a) and 7(b) illustrate a perspective view and a cross-sectional view of a backing element of an ultrasonic probe in accordance with the fourth example.

As illustrated on FIG. 7(a), a backing element 30 of an ultrasonic probe in accordance with the fourth example includes a member 31 having a shape of a polygonal column and a conductive trace 32 formed at one side surface of the member 31. In FIG. 7(a), as the member having a shape of a polygonal column, the member 31 having a shape of a triangular column from various polygonal columns is provided as an example.

The backing element 30 illustrated in FIG. 7(a) is a basic component of a backing layer that is configured to absorb an ultrasonic wave that is radiated toward a rear of the piezo-electric layer by the resonance of a piezo-electric element (oscillator) that composes the piezo-electric layer at an inside of a transducer module. Here, the member 31 having a shape of a polygonal column is manufactured by a synthetic material composed of epoxy resin, tungsten, and rubber powder, and other than the synthetic material. Any material suitable for absorbing rear sound caused by the resonance of piezo-electric material may be used in manufacturing the member 31 having a shape of a polygonal column.

As illustrated in FIG. 7(a), at one side surface of the member 31 having a shape of a triangular column while provided with three units of side surfaces thereof, a conductive trace 32 is formed. Here, the forming of the conductive trace 32 is referred to as the adding (plastering) electrical contact material on a side surface of the member 31 having a shape of a polygonal column by using various adjustment synthesis techniques. When forming the conductive trace 32, as the electrical contact material, metals, conductive graphite, or conductive ink may be used. As a method of adding electrical contact point material on a side surface of the member 31 having a shape of a polygonal column, a technique such as laser scribing, planting, deposition, or printing may be applied.

The backing element 30 is electrically and acoustically connected to one ultrasonic transducer element 40. The ultrasonic transducer element 40 is generally composed of PZT (Lead Zirconate Titanate Ceramics) or a MUT (Micro-machined Ultrasonic Transducer). The electrical and acoustic connection between the backing element 30 and the ultrasonic transducer element 40 will be described in more detail. The member 31 that composes the backing element 30 and has a shape of a polygonal column is acoustically connected to the one ultrasonic transducer element 40 by making contact with the one ultrasonic transducer element 40, so that the member 31 weakens or absorbs acoustic energy that is radiated from a rear surface of the ultrasonic transducer element 40. In addition, the conductive trace 32 formed at one side surface of the member 31 having a shape of a polygonal column is electrically connected to the one ultrasonic transducer element 40, thereby electrically connects the backing element to the ultrasonic transducer element 40.

FIG. 7(b) shows a cross section of the backing element 30, and illustrates an image of the conductive trace 32 formed only at one side surface of the member 11 having a shape of a triangular column provided with three units of side surfaces thereof.

FIG. 8 is a perspective view of a backing layer of the ultrasonic probe formed by arranging the backing elements of the ultrasonic probe in a one-dimensional manner in accordance with the fourth example.

The backing element 30, which is same as the backing element 30 illustrated in FIG 7(a), is provided in plural units (an 'n' number of the backing element 30). By arranging a plurality of backing elements 30a to 30n in a one-dimensional manner, that is, in a linear manner, a backing layer 300 is formed as illustrated on FIG. 8.

As illustrated in FIG. 8, at the backing layer 300 formed by arranging the plurality (the 'n' number) of the backing elements 30a to 30n in a one-dimensional manner, a one-dimensional ultrasonic transducer array 400 formed by arranging a plurality (an 'n' number) of ultrasonic transducer elements 40a to 40n in a one-dimensional manner is electrically and acoustically connected. The electrical and acoustic connection between the backing layer 300 and the one-dimensional ultrasonic transducer array 400 will be described in detail. The members 31a to 31n having a shape of a polygonal column that compose the backing layer 300 are acoustically connected to the one-dimensional ultrasonic transducer array 400 by making contact with the one-dimensional ultrasonic transducer array 400, so that the members weakens or absorbs acoustic energy that is radiated from a rear surface of the one-dimensional ultrasonic transducer array 400. In addition, conductive traces 32a to 32n each formed at each of the members 31a to 31n having a shape of a polygonal column are electrically connected to the plurality (the 'n' number) of the ultrasonic transducer elements that composes the one-dimensional ultrasonic transducer array 400, respectively, thereby electrically connect the members to the ultrasonic transducer elements 40a to 40n. Although not illustrated on FIG. 8, the backing layer 300 of the ultrasonic probe is mounted on a printed circuit board (not shown), and the conductive traces 32a to 32n each formed at each of the members 31a to 31n having a shape of a polygonal column forming the backing layer 300 are electrically connected to conductive wires formed at the printed circuit board, and electrically connect each of the ultrasonic transducer elements 40a to 40n.

FIG. 9 is a perspective view of a backing layer of the ultrasonic probe formed by arranging the backing elements of the ultrasonic probe in a two-dimensional manner in accordance with the fourth example.

The backing element 30, which is same as the backing element 30 illustrated in FIG 7(a), is provided in plural units (an 'n' number of the backing element 30). By arranging a plurality of backing elements 30a to 30n in a two-dimensional manner, that is, in a matrix manner, a backing layer 300 is formed as illustrated on FIG. 9.

As illustrated on FIG. 9, a two-dimensional ultrasonic transducer array 400 formed by arranging a plurality (an 'm' number) of ultrasonic transducer elements 40a to 40m in a two-dimensional manner is electrically and acoustically connected to the backing layer 300 formed by arranging the plurality (the 'n' number) of the backing elements 30a to 30n in a two-dimensional manner. The electrical and acoustic connection between the backing layer 300 and the two-dimensional ultrasonic transducer array 400 will be described in more detail. The plurality of members 31a to 31n having a shape of a polygonal column that compose the backing layer 300 are acoustically connected to the two-dimensional ultrasonic transducer array 400 by making contact with the two-dimensional ultrasonic transducer array 400, so that the plurality of members weakens or absorbs acoustic energy that is radiated from a rear surface of the two-dimensional ultrasonic transducer array 400. In addition, the conductive traces 32a to 32n each formed at each of the plurality of members 31a to 31n having a shape of a polygonal column are electrically connected to the plurality (the 'm' number) of the ultrasonic transducer elements that forms the two-dimensional ultrasonic transducer array 400, respectively, thereby electrically connect the plurality of members to the ultrasonic transducer elements 40a to 40m. Although not illustrated on FIG. 9, the backing layer 300 of the ultrasonic probe is mounted on a printed circuit board (not shown), and the conductive traces 32a to 32n each formed at each of the members 31a to 31n having a shape of a polygonal column forming the backing layer 300 are electrically connected to conductive wires formed at the printed circuit board, and electrically connect each of the ultrasonic transducer elements 40a to 40m.

FIG. 10 illustrates a perspective view of a backing element of an ultrasonic probe in accordance with the fifth example, and a perspective view of a backing layer of the ultrasonic probe formed by arranging the backing elements of the ultrasonic probe in a one-dimensional manner in accordance with the fifth example.

As illustrated in FIG. 10(a), the backing element 30 of the ultrasonic probe in accordance with the fifth example is different from the backing element 30 illustrated in FIG. 7(a). In the fifth example, an electrode layer 33 is further formed at an upper surface or at a lower surface of the member 31 having a shape of a polygonal column acoustically connected to the ultrasonic transducer element 40. As such, since the electrode layer 33 is formed at an upper surface or at a lower surface of the member 31 having a shape of a polygonal column acoustically connected to the ultrasonic transducer element 40, the electrical connection between the ultrasonic transducer element 40 and the conductive trace 32 formed at a side surface of the member 31 having a shape of a polygonal column may be further improved.

The backing element 30, which is same as the backing element 30 illustrated in FIG 10(a), is provided in plural units (an 'n' number of the backing element 30). By arranging a plurality of backing elements 30a to 30n in a one-dimensional manner, that is, in a linear manner, the backing layer 300 is formed as illustrated in FIG. 10(b).

With respect to the structure of a circuit of the backing layer 300 of the ultrasonic probe formed by arranging the backing elements of the ultrasonic probe in a one-dimensional manner in accordance with the fifth example as illustrated in FIG. 10(b), other than the structure of the electrode layer 33 formed at an upper surface or at a lower surface of the member 31 having a shape of a polygonal column, since all other components are same as the structure of the backing layer 300 of the ultrasonic probe formed by arranging the backing elements of the ultrasonic probe in a one-dimensional manner in accordance with the fourth example as illustrated on FIG. 7, the detailed description thereof will be omitted.

FIG. 11A(a) is a cross-sectional view of the backing element 30 having the member 31 having a shape of a triangular column and the conductive trace 32 formed at one side surface of the member 31 having a shape of a triangular column, FIG. 11A(b) is a cross-sectional view of the backing layer 300 formed by arranging the backing elements 30 illustrated at FIG. 11A(a) in a one-dimensional manner, and FIG. 11A(c) is a cross-sectional view of the backing layer 300 formed by arranging the backing elements 30 illustrated at FIG. 11A(a) in a two-dimensional manner. The linear line illustrated with a dotted line on FIGS. 11A(b) and 11A(c) is referred to as a dicing line 'L'. At a domain (a curvature domain illustrated with dotted line at FIG. 11A(c), that is, the "G" domain) divided by the dicing line 'L', one ultrasonic transducer element is connected.

Meanwhile, in the case of forming the backing layer 300 by arranging the members 31 having a shape of a triangular column in a two-dimensional manner while each member 31 is provided with the conductive trace 32 formed only at one side surfaces thereof (refer to FIG. 11A(c)), at each of an in-between area of the arrangements of the members 31 having a shape of a triangular column, an empty space (the "H" domain of FIG. 11A(c)) is formed. The backing layer 300 is needed to perform a function to prevent an image distortion by blocking an ultrasonic wave being proceeded toward a rear of the piezo-electric layer. When an empty space is present at an inside of the backing layer 300, the backing performance of the backing layer 300 may be reduced. Thus, in a case when an empty space is formed between the arrangements of the members having a shape of a triangular column, as in the case of the two-dimensional arrangement of the members 31 having a shape of a triangular column, the plurality of members provided with various shapes of polygonal column thereof may be adhesively attached to each other by using the same material as the material of the members, so that an empty space "H" may be filled with the same material as the material of the members, and thus the valid area of the backing layer 300 may be increased.

The case of the forming of the backing layer 300, which is prepared by arranging the members 31 having a shape of a polygonal column in a one-dimensional manner or in a two-dimensional manner while each of the members 31 having a shape of a polygonal column is provided with the conductive trace 32 formed only at one side surface thereof, has been described. Hereinafter, by referring to FIG. 11B, a case of the forming of the backing layer 300, which is prepared by arranging the members 31 having a shape of a polygonal column in a one-dimensional manner or in a two-dimensional manner while each of the members 31 having a shape of a polygonal column is provided with the conductive traces 32 formed at two side surfaces thereof, will be described.

FIG. 11B(a) is a cross-sectional view of the backing element 30 having the member 31 having a shape of a polygonal (triangular) column and the conductive traces 32 formed at two side surfaces of the member 31 having a shape of a triangular column, FIG. 11B(b) is a cross-sectional view of the backing layer 300 formed by arranging the backing elements 30 illustrated at FIG. 11B(a) in a one-dimensional manner, and FIG. 11B(c) is a cross-sectional view of the backing layer 300 formed by arranging the backing elements 30 illustrated at FIG. 11B(a) in a two-dimensional manner. The linear line illustrated with a dotted line on FIGS. 11B(b) and 11B(c) is referred to as a dicing line 'L'. At a domain (a curvature domain illustrated with dotted line at FIG. 11B(c), that is, the "I" domain) divided by the dicing line 'L', one ultrasonic transducer element is connected.

When comparing the case of forming the backing layer 300 by arranging the members 31 having a shape of a triangular column in a two-dimensional manner while each member 31 is provided with the conductive trace 32 formed only at one side surface thereof (refer to FIG. 11A(c)) to the case of forming the backing layer 300 by arranging the members 31 having a shape of a triangular column in a two-dimensional manner while each member 31 is provided with the conductive traces 12 formed at two side surfaces thereof (refer to FIG. 11B(c)), with respect to the conductive trace 32 present at the "G" domain illustrated in FIG. 11A(c), the conductive trace 32 present at the "I" domain illustrated in FIG. 11B(c) is greater by about four times. As such, since the amount of the conductive trace 32 electrically connected to one ultrasonic transducer element increases, the electrical characteristic of the conductive trace 32 may be further improved.

FIG. 12A(a) shows a cross-sectional view of the backing element 30 that includes the member 31 having a shape of a triangular column and also includes the conductive traces 32 formed at two side surfaces of the member 31 having a shape of a triangular column, and also shows a cross-sectional view of a backing element having a shape of a hexagonal column while being formed by a combination of six of the backing elements 30 of the above, FIG. 12A(b) shows a cross-sectional view of the backing layer 300 formed by arranging the backing elements illustrated at FIG. 12A(a) in a one-dimensional manner while each of the backing elements is provided with a shape of a hexagonal column, and FIG. 12A(c) is a cross-sectional view of the backing layer 300 formed by arranging the backing elements 30 illustrated at FIG. 12A(a) in a two-dimensional manner while each of the backing elements is provided with a shape of a hexagonal column. At a curvature domain (a domain illustrated with a dotted line at FIG. 12A(c), that is, a "J" domain), one ultrasonic transducer element is connected.

When comparing the case of forming the backing layer 300 by arranging the members 31 having a shape of a triangular column in a two-dimensional manner while each of the members 31 is provided with the conductive trace 32 formed only at one side surface thereof (refer to FIG. 11A(c)) to the case of forming the backing layer 300 (refer to FIG. 12A(c)) by arranging the backing elements each formed by a combination of six of the backing elements 30 each having a shape of a triangular column while the backing element is provided with a shape of a hexagonal column (refer to FIG. 12A(a)), with respect to the conductive trace 32 present at the "G" domain illustrated on FIG. 11A(c), the conductive trace 32 present at the "J" domain illustrated on FIG. 12A(c) is greater by about twelve times. As such, since the amount of the conductive trace 32 electrically connected to one ultrasonic transducer element increases, the electrical characteristic of the conductive trace 32 may be further improved.

FIG. 12B(a) shows a cross-sectional view of the backing element 30 that includes the member 31 having a shape of a triangular column and also includes the conductive traces 32 formed at two side surfaces of the member 31 having a shape of a triangular column, and also shows a cross-sectional view of a backing element having a shape of an octagonal column while being formed by a combination of eight of the backing elements 30 of the above, FIG. 12B(b) is a cross-sectional view of the backing layer 300 formed by arranging the backing elements illustrated at FIG. 12B(a) in a one-dimensional manner while each of the backing elements is provided with a shape of an octagonal column, and FIG. 12B(c) is a cross-sectional view of the backing layer 300 formed by arranging the backing elements 30 illustrated at FIG. 12B(a) in a two-dimensional manner while each of the backing elements is provided with a shape of an octagonal column. At a curvature domain (a domain illustrated with a dotted line at FIG. 12B(c), that is, a "K" domain), one ultrasonic transducer element is connected.

When comparing the case of forming the backing layer 300 by arranging the members 31 having a shape of a triangular column in a two-dimensional manner while each of the members 31 is provided with the conductive trace 32 formed only at one side surface thereof (refer to FIG. 11A(c)) to the case of forming the backing layer 300 (refer to FIG. 12B(c)) by arranging the backing element formed by a combination of the eight backing elements 30 each having a shape of a triangular column while the backing element is provided with a shape of an octagonal column (refer to FIG. 12B(a)), with respect to the conductive trace 32 present at the "G" domain illustrated in FIG. 11A(c), the conductive trace 32 present at the "K" domain illustrated in FIG. 12B(c) is greater by about sixteen times. As such, since the amount of the conductive trace 32 electrically connected to one ultrasonic transducer element increases, the electrical characteristic of the conductive trace 32 may be further improved.

Meanwhile, in the case of forming the backing layer 300 by arranging the backing element in a two-dimensional manner while the backing element is formed by a combination of the eight backing elements 30 each having a shape of a triangular column (refer to FIG. 12B(c)), at each of an in-between area of the arrangements of the backing element each having a shape of an octagonal column, an empty space (an "M" domain of FIG. 12B(c)) is formed. The backing layer 300 is needed to perform a function to prevent an image distortion by blocking an ultrasonic wave being proceeded toward a rear of the piezo-electric layer. When an empty space is present at an inside of the backing layer 300, the backing performance of the backing layer 300 may be reduced. Thus, in addition to a case when an empty space is formed between the arrangements of the backing elements each having a shape of an octagonal column, as in the case of the two-dimensional arrangement of the backing elements each having a shape of an octagonal column, also in a case when a visible empty space is not formed at each of an in-between of the arrangements of the backing elements each having a shape of a hexagonal column, as in the case of the two-dimensional arrangement of the backing elements each having a shape of a hexagonal column, the plurality of members provided with various shapes of polygonal column thereof may be adhesively attached to each other by using the same material as the material of the members when arranging the plurality of members provided with various shapes of polygonal column thereof in a one-dimensional manner or in a two-dimensional manner, and thus the valid area of the backing layer 300 may be increased.

In the present disclosure, examples of forming a backing layer by arranging members each having a shape of a triangular column, a hexagonal column and an octagonal column in a one-dimensional manner or in a two-dimensional manner while each member is provided with a conductive trace formed at one side surface or at two or more side surfaces thereof have been described. However, in an example not forming part of the invention, the forming of a backing layer may be possible by arranging members each having various shapes of a polygonal column (for example, a dodecagonal column) other than the shapes above in a one-dimensional manner or in a two-dimensional manner.

In addition, in the present disclosure, examples of forming a backing layer by arranging members having a single shape of a polygonal column (for example, a shape of a triangular column, a shape of a hexagonal column, or a shape of an octagonal column) in a one-dimensional manner or in a two-dimensional manner have been described. However, in an example not forming part of the invention, the forming of a backing layer may be possible by combining a one shape of a polygonal column with the other shape of a polygonal column (for example, a combination of the members each having a shape of an octagonal column and the members each having a shape of a rectangular column).

Although a few embodiments of the present disclosure have been shown and described, it would be appreciated by those skilled in the art that changes may be made in these embodiments without departing from the principles of the disclosure, the scope of which is defined in the claims.

## Claims

1. An ultrasonic probe comprising:
an ultrasonic transducer array (200) comprising a plurality of ultrasonic transducer elements (20); and
a backing layer (100) acoustically connected to the ultrasonic transducer array (200), comprising a plurality of backing elements (10), which are configured to be arranged in a two-dimensional manner, **characterized in that** each backing element (10) comprises:
a member (11) having a shape of a hexagonal column acoustically connected to the plurality of ultrasonic transducer elements (20);
two conductive traces (12) disposed diametrically opposed at two side surfaces of the member (11); and
wherein one of the two conductive traces (12) is electrically connected to one of the plurality of ultrasonic transducer elements (20) and the other one of the two conductive traces (12) is electrically connected to another one of the plurality of ultrasonic transducer elements (20).

2. An ultrasonic probe comprising:
an ultrasonic transducer array (200) comprising a plurality of ultrasonic transducer elements (20); and
a backing layer (100) acoustically connected to the ultrasonic transducer array (200), comprising a plurality of backing elements (10), which are configured to be arranged in a two-dimensional manner, **characterized in that** each backing element (10) comprises:
a member (11) having a shape of an octagonal column acoustically connected to the plurality of ultrasonic transducer elements (20);
four conductive traces (12) disposed at every second side surface of the member (11); and
wherein one of the four conductive traces (12) is electrically connected to one of the plurality of ultrasonic transducer elements (20) and another one of the four conductive traces (12) is electrically connected to another one of the plurality of ultrasonic transducer elements (20).

3. The ultrasonic probe of claim 1 or 2, wherein:
one of the upper surface and the lower surface of each of the plurality of members (11) is acoustically connected to the ultrasonic transducer array (200).

4. The ultrasonic probe of claim 1 or 2, wherein:
the plurality of members (11) are adhesively attached to each other by using a same material as a material of the plurality of members (11).

5. A method of manufacturing an ultrasonic probe, comprising an ultrasonic transducer array (200) comprising a plurality of ultrasonic transducer elements (20) and a backing layer (100) comprising a plurality of backing elements (10), which are configured to be arranged in a two-dimensional manner, each backing element comprising a backing member and conductive traces, the method comprising the steps of:
preparing a plurality of backing members (11) each having a shape of a hexagonal column;
arranging the plurality of backing members (11) in a two-dimensional manner;
acoustically connecting the plurality of backing members (11) to the plurality of ultrasonic transducer elements (20); and
disposing two conductive traces (12) diametrically opposed at two side surfaces of each member; wherein one of the two ef- conductive traces (12) is electrically connected to one of the plurality of ultrasonic transducer elements (20) and the other one of the two conductive traces (12) is electrically connected to another one of the plurality of ultrasonic transducer elements (20).

6. A method of manufacturing an ultrasonic probe, comprising an ultrasonic transducer array (200) comprising a plurality of ultrasonic transducer elements (20) and a backing layer (100) comprising a plurality of backing elements (10), which are configured to be arranged in a two-dimensional manner, each backing element comprising a backing member and conductive traces, the method comprising the steps of:
preparing a plurality of backing members (11) each having a shape of an octagonal column;
arranging the plurality of backing members (11) in a two-dimensional manner;
acoustically connecting the plurality of backing members (11) to the plurality of ultrasonic transducer elements (20) of an ultrasonic transducer array (200); and
disposing four conductive traces (12) at every second side surface of each member;
wherein one of the four
conductive traces (12) is electrically connected to one of the plurality of ultrasonic transducer elements (20) and another one of the four conductive traces (12) is electrically connected to another one of the plurality of ultrasonic transducer elements (20).

## Patentansprüche

1. Ultraschallsonde, welche Folgendes aufweist:
eine Ultraschallumwandleranordnung (200), die eine Vielzahl von Ultraschallumwandlerelementen (20) aufweist; und
eine Trägerschicht (100), die mit der Ultraschallumwandleranordnung (200) akustisch verbunden ist und eine Vielzahl von Trägerelementen (10) aufweist, die dafür vorgesehen sind, in einer zweidimensionalen Art und Weise angeordnet zu sein,
**dadurch gekennzeichnet, dass**
jedes Trägerelement (10) Folgendes aufweist:
ein Element (11) mit einer Form einer hexagonalen Säule, das akustisch mit der Vielzahl von Ultraschallumwandlerelementen (20) verbunden ist;
zwei Leiterbahnen (12), die einander diametral gegenüberliegend an zwei Seitenflächen des Elements (11) angeordnet sind; und
wobei eine der zwei Leiterbahnen (12) elektrisch mit einem der Vielzahl von Ultraschallumwandlerelementen (20) elektrisch verbunden ist und die andere der zwei Leiterbahnen (12) elektrisch mit einem anderen der Vielzahl von Ultraschallumwandlerelementen (20) elektrisch verbunden ist.

2. Ultraschallsonde, welche Folgendes aufweist:
eine Ultraschallumwandleranordnung (200), die eine Vielzahl von Ultraschallumwandlerelementen (20) aufweist; und
eine Trägerschicht (100), die mit der Ultraschallumwandleranordnung (200) akustisch verbunden ist und eine Vielzahl von Trägerelementen (10) aufweist, die dafür vorgesehen sind, in einer zweidimensionalen Art und Weise angeordnet zu sein,
**dadurch gekennzeichnet, dass**
jedes Trägerelement (10) Folgendes aufweist:
ein Element (11) mit einer Form einer hexagonalen Säule, das akustisch mit der Vielzahl von Ultraschallumwandlerelementen (20) verbunden ist;
vier Leiterbahnen (12), die an jeder zweiten Seitenfläche des Elements angeordnet sind; und
wobei eine der vier Leiterbahnen (12) mit einem der Vielzahl von Ultraschallumwandlerelementen (20) elektrisch verbunden ist und eine weitere der vier Leiterbahnen (12) mit einem anderen der Vielzahl von Ultraschallumwandlerelementen (20) elektrisch verbunden ist.

3. Ultraschallsonde nach Anspruch 1 oder 2, wobei:
eine der oberen Fläche und der unteren Fläche von jedem der Vielzahl von Elementen (11) mit der Ultraschallumwandleranordnung (200) akustisch verbunden ist.

4. Ultraschallsonde nach Anspruch 1 oder 2, wobei:
die Vielzahl von Elementen (11) unter Verwendung desselben Materials wie ein Material der Vielzahl von Elementen (11) anhaftend aneinander angebracht sind.

5. Verfahren zur Herstellung einer Ultraschallsonde, die eine Ultraschallumwandleranordnung (200) mit einer Vielzahl von Ultraschallumwandlerelementen (20) und einer Trägerschicht (100) mit einer Vielzahl von Trägerelementen (10) aufweist, die dafür vorgesehen sind, auf eine zweidimensionale Art und Weise angeordnet zu sein, wobei jedes Trägerelement ein Trägerelement und Leiterbahnen aufweist, wobei das Verfahren die folgenden Schritte aufweist:
Vorbereiten einer Vielzahl von Trägerelementen (11), die jeweils eine Form einer hexagonalen Säule aufweisen;
Anordnen der Vielzahl von Trägerelementen (11) auf eine zweidimensionale Art und Weise;
akustisches Verbinden der Vielzahl von Trägerelementen (11) mit der Vielzahl von Ultraschallumwandlerelementen (20); und
Anordnen zweier Leiterbahnen (12) einander diametral gegenüberliegend an zwei Seitenflächen von jedem Element, wobei eine der zwei Leiterbahnen (12) elektrisch mit einem der Vielzahl von Ultraschallumwandlerelementen (20) verbunden wird und die andere der zwei Leiterbahnen (12) mit einem anderen der Vielzahl von Ultraschallumwandlerelememten (20) elektrisch verbunden wird.

6. Verfahren zur Herstellung einer Ultraschallsonde, die eine Ultraschallumwandleranordnung (200) mit einer Vielzahl von Ultraschallumwandlerelementen (20) und einer Trägerschicht (100) mit einer Vielzahl von Trägerelementen (10) aufweist, die dafür vorgesehen sind, auf eine zweidimensionale Art und Weise angeordnet zu sein, wobei jedes Trägerelement ein Trägerelement und Leiterbahnen aufweist, wobei das Verfahren die folgenden Schritte aufweist:
Vorbereiten einer Vielzahl von Trägerelementen (11), die jeweils eine Form einer hexagonalen Säule aufweisen;
Anordnen der Vielzahl von Trägerelementen (11) auf eine zweidimensionale Art und Weise;
akustisches Verbinden der Vielzahl von Trägerelementen (11) mit der Vielzahl von Ultraschallumwandlerelementen (20) einer Ultraschallumwandleranordnung (200); und
Anordnen von vier Leiterbahnen (12) an jeder zweiten Seitenfläche von jedem Element;
wobei eine der vier Leiterbahnen (12) mit einem der Vielzahl von Ultraschallumwandlerelementen (20) elektrisch verbunden wird und eine andere der vier Leiterbahnen (12) mit einem anderen der Vielzahl von Ultraschallumwandlerelememten (20) elektrisch verbunden wird.

## Revendications

1. Sonde à ultrasons comprenant :
un réseau de transducteurs ultrasonores (200) comprenant une pluralité d'éléments transducteurs ultrasonores (20) ; et
une couche de soutien (100) reliée acoustiquement au réseau de transducteurs ultrasonores (200), comprenant une pluralité d'éléments de soutien (10), qui sont configurés pour être agencés d'une manière bidimensionnelle, **caractérisée en ce que** chaque élément de soutien (10) comprend :
un élément (11) ayant une forme d'une colonne hexagonale relié acoustiquement à la pluralité d'éléments transducteurs ultrasonores (20) ;
deux pistes conductrices (12) disposées diamétralement opposées sur deux surfaces latérales de l'élément (11) ; et
dans laquelle l'une des deux pistes conductrices (12) est reliée électriquement à l'un de la pluralité d'éléments transducteurs ultrasonores (20) et l'autre des deux pistes conductrices (12) est reliée électriquement à un autre de la pluralité d'éléments transducteurs ultrasonores (20).

2. Sonde à ultrasons comprenant :
un réseau de transducteurs ultrasonores (200) comprenant une pluralité d'éléments transducteurs ultrasonores (20) ; et
une couche de soutien (100) reliée acoustiquement au réseau de transducteurs ultrasonores (200), comprenant une pluralité d'éléments de soutien (10), qui sont configurés pour être agencés d'une manière bidimensionnelle, **caractérisée en ce que** chaque élément de soutien (10) comprend :
un élément (11) ayant une forme d'une colonne octogonale relié acoustiquement à la pluralité d'éléments transducteurs ultrasonores (20) ;
quatre pistes conductrices (12) disposées au niveau de chaque seconde surface latérale de l'élément (11) ; et
dans lequel l'une des quatre pistes conductrices (12) est reliée électriquement à l'un de la pluralité d'éléments transducteurs ultrasonores (20) et une autre des quatre pistes conductrices (12) est reliée électriquement à un autre de la pluralité d'éléments transducteurs ultrasonores (20).

3. Sonde à ultrasons selon la revendication 1 ou 2, dans laquelle :
l'une de la surface supérieure et de la surface inférieure de chacun de la pluralité d'éléments (11) est reliée acoustiquement au réseau de transducteurs ultrasonores (200).

4. Sonde à ultrasons selon la revendication 1 ou 2, dans laquelle :
la pluralité d'éléments (11) sont fixés par adhésif les uns aux autres en utilisant un même matériau qu'un matériau de la pluralité d'éléments (11).

5. Procédé de fabrication d'une sonde à ultrasons, comprenant un réseau de transducteurs ultrasonores (200) comprenant une pluralité d'éléments transducteurs ultrasonores (20) et une couche de soutien (100) comprenant une pluralité d'éléments de soutien (10), qui sont configurés pour être agencés d'une manière bidimensionnelle, chaque élément de soutien comprenant un élément de soutien et des pistes conductrices, le procédé comprenant les étapes consistant à :
préparer une pluralité d'éléments de soutien (11) ayant chacun une forme d'une colonne hexagonale ;
agencer la pluralité d'éléments de soutien (11) d'une manière bidimensionnelle ;
relier acoustiquement la pluralité d'éléments de soutien (11) à la pluralité d'éléments transducteurs ultrasonores (20) ; et
disposer deux pistes conductrices (12) diamétralement opposées sur deux surfaces latérales de chaque élément ; dans lequel l'une des deux pistes conductrices (12) est reliée électriquement à l'un de la pluralité d'éléments transducteurs ultrasonores (20) et l'autre des deux pistes conductrices (12) est reliée électriquement à un autre de la pluralité d'éléments transducteurs ultrasonores (20).

6. Procédé de fabrication d'une sonde à ultrasons, comprenant un réseau de transducteurs ultrasonores (200) comprenant une pluralité d'éléments transducteurs ultrasonores (20) et une couche de soutien (100) comprenant une pluralité d'éléments de soutien (10), qui sont configurés pour être agencés d'une manière bidimensionnelle, chaque élément de soutien comprenant un élément de soutien et des pistes conductrices, le procédé comprenant les étapes consistant à :
préparer une pluralité d'éléments de soutien (11) ayant chacun une forme d'une colonne hexagonale ;
agencer la pluralité d'éléments de soutien (11) d'une manière bidimensionnelle ;
relier acoustiquement la pluralité d'éléments de soutien (11) à la pluralité d'éléments transducteurs ultrasonores (20) d'un réseau de transducteurs ultrasonores (200) ; et
disposer quatre pistes conductrices (12) au niveau de chaque seconde surface latérale de chaque élément ;
dans lequel l'une des quatre pistes conductrices (12) est reliée électriquement à l'un de la pluralité d'éléments transducteurs ultrasonores (20) et une autre des quatre pistes conductrices (12) est reliée électriquement à un autre de la pluralité d'éléments transducteurs ultrasonores (20).
